# EUROPEAN PATENT APPLICATION

(11) **EP 1 974 661 A1**
(43) Date of publication of application: **01.10.2008**
(21) Application number: 08153389.5
(22) Date of filing: 27.03.2008
(51) Int. Cl.: A61B 5/00, G09B 7/00, G09B 23/28, G06F 19/00

(54) **An accurate method to assess disease severity in clinical trials concerning psychopathology**

(30) Priority: 28.03.2007 EP 07105155
(71) Applicant: N.V. Organon, 5349 AB Oss (NL)
(72) Inventor: Schoemaker, Josephus Hubertus, 5340 BH, Oss (NL)
(74) Representative: Broekkamp, Chris L. E.

(57) **Abstract**

The invention provides for an accurate method to assess disease severity in clinical trials involving psychopathology, which method comprises the sequence of a) making audiovisual, identified and patient-observer-linked recordings of interviews between observers and patients; c) presenting the identified recordings for review to one or more reference observers; d) asking the reference observer or observers to score the disease or symptom severity with the said rating scale on basis of observation of the identified recordings; e) making the scores based on the patient-observer recordings by the reference observer or reference observers available to the corresponding observers; whereby the identified recordings are made of interviews held by the observers with their patients for producing the scores by the observers for the assessment of the effect of the intervention and the method comprises in the sequence the step: f) asking the observers to consolidate or adjust the scores to be used for obtaining the overall score.

## Description

The invention relates to a method to assess severity of a disease in a group of patients by combining measurements from a plurality of observers, each of which provides a score of the disease severity in a patient at a particular point in time with a rating scale, and which scores are pooled to obtain an overall score of the group.

A common problem in randomized clinical trials in the central nervous system therapeutic area is a failure to demonstrate that subjects do better on active drugs than on placebo. This is caused by an unfavorable signal/noise ratio resulting from one or more of following factors:
1. Inaccurate patient diagnosis or, more in general, non-adherence to the study in-/exclusion criteria;
2. Differences in drop-out rates across study arms;
3. Bias, or erroneous scores, due to
   3.1. Inflated or deflated baseline scores to fulfill study entry criteria and subsequent 'normalization' of rating scale scores in placebo-treated subjects, suggesting response to treatment (References 1, 2);
   3.2. Investigator and/or subject expectations regarding (double-blind, experimental) treatment (References 3, 4, 5, 6).
   3.3. Financial interests of investigator and/or patient to lengthen trial participation;
   3.4. Guessing of treatment group based on presence or absence of adverse events;
   3.5. Learning effects through repeated questioning of patients with the same rating scale;
   3.6. Reliance on rapport of subjects who may lack insight in own illness or basic understanding of questions;
   3.7. Poor rating interview skills or limited compliance with rating guidelines;
4. Inter-rater variability (inconsistent scoring of symptom severity), due to
   4.1. Differences in clinical experience (Reference 7),
   4.2. Differences in education and training (Reference 7),
   4.3. Differences in interview techniques / skills (Reference 8, 9 and 10),
   4.4. Differences in cultural background,
   4.5. Differences in language skills,
   4.6. Differences in rating scale versions,
   4.7. Non-adherence to rating rules and conventions,
   4.8. Rater drift and/or turnover;
5. Non compliance with treatment;
6. Chronicity, comorbidity, and/or low baseline level of disease severity, allowing only minimal improvement over time;

Following measures may be taken to minimize the influence of above factors and inherent risk for failed clinical trials:
Ad 1) Use of a diagnostic interview schedule, per protocol analysis (excluding subjects with protocol violations from the efficacy analysis), conservative enrolment forecasts, and /or more explicit definition of critical eligibility criteria.
Ad 2) Assessment of efficacy according to so-called Mixed-Model-Repeated-Measurements method.
Ad 3) Centralized (independent) ratings or averaging (or consensus) scores of more than one rater per interview (3.1-4), offering of rescue treatment for dropouts or financial compensation made independent of trial duration (3.3), minimization of assessment frequencies (3.5), primary analysis focused on investigator reported outcome and consideration of information about the patient from caregivers and relatives in the overall assessment of disease severity (3.6), concurrent assessment of rater interview performance and subsequent exclusion of scores from raters not meeting quality criteria for adequate interview techniques (3.7).
Ad 4)
   o Only allow investigators with minimum level of experience (4.1) and/or education (4.2) in the trial;
   o Implementation of a rater training program with certification (4.2, 4.3, 4.4), in-study refresher trainings (re-certification), limitation of trial duration and web-based trainings allowing new raters to qualify during the trial (4.8), retrospective (statistical) confirmation of sufficient level of inter-rater reliability (4.1-5), and in-study tutorials based on ratings reliability assessments (4.1-5, 4.8);
   o Use of a scale-specific, (semi-)structured clinical interview schedule (4.3);
   o Use of a single rating scale version (4.6) and validated translations (4.5);
Ad 5) Patient education, obligatory hospitalization, or use of other compliance enhancement methods or techniques, such as medication dispensers with alarm clock.
Ad 6) Inclusion of only acutely, and moderately to severely ill patients with no intake of concomitant medication;

Some prior art, in which video recordings of patient-rater interviews can be used, provide for training methods as indicated above (Ad 4, second bullet point), e.g. US 2003/125610 (Sachs et al) and US 2004/015329 (Shayega et al). In such training methods the rater can correct (remediate) their judgment methods. Once sufficiently trained such persons can act as certified raters in clinical trials.

Despite the fact that the above measures are commonly taken by sponsors of clinical trials in the area of central nervous system (CNS) diseases, the failure rate of these trials may still be up to 50% in some indications.

Applied clinical skills remain difficult to monitor and to control during a clinical trial. A recent study finds a significant relationship between interview quality and signal detection (Reference 10)

The present invention provides for a method that augments the quality of patient assessments and inter-rater reliability, thus enhancing signal detection in CNS clinical trials. The new method involves the use of existing techniques but in a unique combination.

The conventional approach (according to the latest state of the art) to detect differences in treatment outcome within the conduct of randomized clinical trials in CNS can be summarized as follows.

At various time points during a clinical study, investigators see patients to assess disease severity on a rating scale. Scores are entered into a Case Report Form (CRF) and used for the analysis of efficacy, comparing the scores at different study time points across treatment arms. Before being allowed to participate in the trial as investigator, raters have to fulfil minimum requirements for education and experience, undergo a dedicated training program (web-based or during an investigator meeting at the start of the trial), and qualify as rater for the clinical study. If the trial duration is relatively long, re-certification may be required at repeated intervals. Raters can be either located at the site, travel from site to site to interview subjects, interview subjects remotely (e.g. through webcam), or assess disease severity on the basis of (audio-) visual recordings of interviews. An essential characteristic of the conventional approach is that the CRF entries are regarded as source document and not further questioned by the study sponsor. In some cases, more than one rater assesses disease severity in an individual patient at a particular time point and the mean of scores or consensus score is used as outcome variable.

The present invention provides a method to assess severity of a disease, for example a psychiatric or neurological disorder, in a group of patients by combining measurements from a plurality of observers, each of which provides a score of the disease severity, or the severity of a symptom thereof, in a patient at a particular point in time with a rating scale, and which scores are pooled to obtain an overall score of the group,
which method comprises the sequence of
a) making audiovisual, identified and patient-observer-linked recordings of interviews between observers and patients;
c) presenting the identified recordings for review to one or more reference observers;
d) asking the reference observer or observers to score the disease or symptom severity with the said rating scale on basis of observation of the identified recordings;
e) making the scores based on the patient-observer recordings by the reference observer or reference observers available to the corresponding observers,
   whereby the identified recordings are made of interviews held by the observers with their patients for producing the scores for the assessment of the effect of the intervention;
   and the method comprises in the sequence the step:
f) asking the observers to consolidate or adjust the scores to be used for obtaining the overall score.
   The method according to the invention can be briefly referred to as Expert Rater Assisted Score Evaluation (ERASE).
   With the method according to the invention, investigators (also the terms 'observers' and 'raters' are used in this specification) are required to make audiovisual recordings of their interaction or interview with patients, allowing reference observers, also referred to herein as expert raters, to assess disease severity after review of the recordings. It is preferred that the expert rater is fluent in the language of the investigator and patient, is thoroughly familiar with the rating instruments, and is not affiliated with the study site. The expert rater scores are made accessible to investigators, who can consolidate or adjust their own scores, or adopt the expert rater scores for individual rating scale items. Also, and at the same time, expert raters can contact investigators when interactions or interviews are inadequate and individual scale items can not be rated. The final (adjusted or consolidated) investigator scores are used as primary outcome variable for the efficacy analysis. This aspect of the invention, that is the provision of a protocol that allows re-adjustment of the rater-scores during the trial (before the definitive rater-scores are stored in the database and the blind is broken) is an important aspect of this invention and a novel aspect over the prior art, for example US 2003/125610 (Sachs et al) and US 2004/015329 (Shayega et al). The investigators are considered to be more knowledgeable about their patients, whereas the expert raters are considered to be experts regarding assessment or interview techniques and scoring conventions. This system of second opinion and feed-back to investigators is providing the following advantages over traditional methods of rating subject at clinical sites:
   - consistently applied assessment or interview techniques,
   - improved inter and intra-investigator reliability,
   - more focussed investigator performance,
   - enhancement of signal detection through increased reliability of the primary outcome variable.
   - Improved data integrity according to pre-defined conventions.

In order to avoid that investigators will await the reference observers scores before entering their own scores on the CRF, audiovisual recordings can be made available for review to the reference observers only after transmission of preliminary investigator scores from the site to a central fax server. Expert raters are kept blind to the primary investigator scores in order to avoid bias, but will receive collateral information from investigators about the patient that is relevant to judge symptom severity on all rating scale items. The audiovisual recordings are confidential material, not made available to the study sponsor, and destroyed after completion of the clinical trial.

In an important aspect the invention provides an accurate assessment of both verbal and non-verbal expression of symptoms, such as poor eye contact with the interviewer, passive social avoidance, inappropriate affect, blunted affect, etc. The improved inter-rater reliability with ERASE is not based on investigator qualification and remediation (i.e. repeat training) but on enforcement of adequate interview techniques and well-founded, or motivated (defendable) interpretation of a patient's verbal and non-verbal expressions.

A further important aspect of the invention is that the site rater or local investigator, who is responsible for treatment of the subject, is considered to have 'expert knowledge' about the patient and is in the best position to score symptom severity most accurately. Video recordings are made of live interviews, and Independent Rater scores are collected during the trial and made accessible to the Site Raters as 'second opinion'. If there is a meaningful discrepancy in symptom scores from the Independent and Site Rater, the latter may revise his/her own scores a posteriori, if considered appropriate, or should otherwise explain why (s)he differs in his/her judgment from the Independent Rater.

Through a system of audiovisual interview recordings at critical time points, repeated feed-back from Independent Raters and individual item score review, Site Raters are expected to provide more reliable symptom scores for all subjects in a clinical trial. According to the method of the invention Site Raters are forced to record adequately conducted interviews that will allow external reviewers to score symptom severity at critical time points during the clinical trial. It is a method that automatically monitors and controls the interview quality of the investigators, assuming that for the accurate scoring of symptom severity the right questions must be asked first of all.

The method according to the invention is not only addressing the aim to enhance inter-rater reliability while monitoring and interpreting patient feedback, but also controls for potential rater drift or poor inter-rater reliability due to non-adherence to appropriate interview standards during the trial. Nevertheless, with the method according to the invention the primary efficacy analysis remains based upon the Site Rater interviews and their (adjusted)scores. The responsibility of evaluating patients is not taken away from the Site Raters, nor is their final evaluation of symptom severity taken into doubt.

Rating scales which can be suitably used in the method are for example the Scale of the Assessment of Negative Symptoms (SANS); Positive and Negative Syndrome Scale (PANSS); the Brief Psychiatric Rating Scale (BPRS); the Hamilton rating scale for depression (HAMD), etc.

### Example

Rating scale assessments by the investigator will be collected from each patient. A centrally located, independent rater fluent in the language of the investigator and patient, and who is thoroughly familiar with the rating instruments, will also be asked to score some of the interviews for each patient throughout the trial. Upon completion, central rater scores will be made available to the investigator, who can compare these with his/her own scores before submitting results to the sponsor of the clinical trial.

Study site personnel will make audiovisual recordings of the baseline and endpoint interviews for the primary efficacy measure, the SANS, as well as the screening interview for the PANSS, using equipment provided by a central rating service provider. Recordings and relevant, collateral information about the subject that is obtained elsewhere (e.g. from caregivers), and considered by the investigator as typically required for an appropriate rating, will be uploaded to a secure web site. A designated central rater, blind to the site ratings, will review and score the patient interview and post his/her scores to the web site. If insufficient information is available to score a particular item, the central rater will record this item as 'not done', and will follow-up by telephone with the investigator to further clarify.

A project specialist of the central service provider will ensure that interview recordings, collateral information, and central rater scores are available at the web site for review by the investigators (restricted by study site) until study close-out. Investigators will be permitted access to the central rater scores at the web site provided they have successfully transferred readable photocopies of completed CRF pages with preliminary site rater scores to the central rating service provider. The site investigator is asked to enter the independent ratings of the expert rater (reference observer) in a CRF. When discrepancies between the (preliminary) scores of an investigator and central rater are meaningful (≥2 points difference in item score) and follow a consistent pattern across different interviews, a remediation tutorial may be provided to the investigator to clarify difficult rating situations and/or to reinforce training on scoring rules and conventions.

The investigator is allowed to incorporate some or all of the central rater scores in his/her own scoring before original CRF pages are submitted to the trial sponsor. A comment from the investigator is requested in the CRF when a meaningful discrepancy (≥2 points difference in item score) exists between the central rater scores and final investigator ratings of SANS global items of PANSS positive, negative and general subscale items.

The sponsor's clinical research scientist and all clinical research associates will have access to all central rater scores at the website but not to any of the interview recordings. Upon database lock, the project specialist will ensure that the web site goes down and that all interview recordings and collateral information are permanently destroyed. Preliminary site rater scores and independent scores are transmitted from the central rating provider to the sponsor for permanent storage into the trial database.

Detailed instructions for recordings, data processing, and criteria for meaningful discrepancies are provided in a separate rater manual. Demonstration of the enablement of the invention is done in this trial comprising 720 interviews.

References:
1. DeBrota, D. J., Demitrack, M. A., Landin, R., Kobak, K. A., Greist, J. H., & Potter, W. Z. (June, 1999). A Comparison Between Interactive Voice Response System-Administered HAM-D and Clinician-Administered HAM-D in Patients with Major Depressive Episode. National Institute of Mental Health, New Clinical Drug Evaluation Unit, presented at 39th Annual Meeting, Boca Raton, FL.
2. Feltner, D. E., Kobak, K. A., Crockatt, J., Haber, H., Kavoussi, R., Pande, A., & Greist, J. H. (May, 2001). Interactive Voice Response (IVR) for Patient Screening of Anxiety in a Clinical Drug Trial. National Institute of Mental Health, New Clinical Drug Evaluation Unit, presented at 41 st Annual Meeting, Phoenix, AZ.
3. Glaudin V, Smith W, Ferguson J, DuBoff E, Rosenthal M, Mee-Lee D. Discriminating placebo and drug in generalized anxiety disorder (GAD) trials: single vs. multiple raters. Psychopharmacology Bulletin. 1994;32(2):175-178.
4. Quinn J, Moore M, Benson DF, Clark CM, Doody R, Jagust W, Knopman D, Kaye JA. A videotaped CIBIC for dementia patients: validity and reliability in a simulated clinical trial. Neurology 2002;58:433-437.
5. DeBrota D, Gelwicks S, Potter W. Same rater versus different raters in depression clinical trials. Presented at 42nd Annual Meeting, New Clinical Drug Evaluation Unit. Boca Raton, FL, June 2002.
6. Kobak KA, Lipsitz JD, Williams JBW, Engelhardt N, Bellew KM. A new approach to rater training in a multicenter clinical trial. Journal of Clinical Psychopharmacology 2005;25:407-412.
7. Kobak KA. Relationship between education, years experience, and clinical competence in depression rating skills. Presented at Drug Information Association, 42nd Annual Meeting, Philadelphia, PA, June 2006.
8. Feiger A, Engelhardt N, DeBrota D, Cogger K, Lipsitz J, Sikich D, Kobak KA, Rating the raters: an evaluation of audio taped Hamilton Depression Rating Scale (HAMD) interviews. Presented at National Institute of Mental Health, New Clinical Drug Evaluation Unit, 43rd Annual Meeting, Boca Raton, Florida (US).
9. Joffe et al. Antidepressant treatment of depression: a meta-analysis. Can J Psychiatry 2006;41:613-616.
10. Kobak, K.A., Feiger A., Lipsitz J. Interview Quality and Signal Detection in Clinical Trials. American Journal of Psychiatry 2005;162:628.
11. Sachs et al US 2003/125610
12. Shayegan et al US 2004/015329

## Claims

1. A method to assess severity of a disease in a group of patients by combining measurements from a plurality of observers, each of which provides a score of the disease severity, or the severity of a symptom thereof, in a patient at a particular point in time with a rating scale, and which scores are pooled to obtain an overall score of the group,
which method comprises the sequence of
a) making audiovisual, identified and patient-observer-linked recordings of interviews between observers and patients;
c) presenting the identified recordings for review to one or more reference observers;
d) asking the reference observer or observers to score the disease or symptom severity with the said rating scale on basis of observation of the identified recordings;
e) making the scores based on the patient-observer recordings by the reference observer or reference observers available to the corresponding observers;
**characterised in that** the identified recordings are made of interviews held by the observers with their patients for producing the scores by the observers for the assessment of the effect of the intervention; and the method comprises in the sequence the step:
f) asking the observers to consolidate or adjust the scores to be used for obtaining the overall score.

2. The method according to claim 1, **characterised in that** the disease is a psychiatric or neurological disorder.
